# FASCICULE DE BREVET EUROPEEN

(11) **EP 1 018 930 B1**
(45) Date de publication et mention de la délivrance du brevet: **11.09.2002**
(21) Numéro de dépôt: 98943623.3
(22) Date de dépôt: 29.09.1998
(51) Int. Cl.: A61B 3/16

(54) **INSTRUMENT DE MESURE DE LA PRESSION OCULAIRE**
AUGENDRUCKMESSGERÄT
INSTRUMENT FOR MEASURING INTRAOCULAR PRESSURE

(30) Priorité: 29.09.1997 CH 239097; 01.03.1998 CH 48798
(43) Date de publication de la demande: 19.07.2000
(73) Titulaire: Heyraud, Marc, 2300 La Chaux-de-Fonds (CH); Grounauer, Pierre-Alain, 1036 Sullens (CH); Kureth, Jean-Bernard, 2300 La Chaux-de-Fonds (CH)
(72) Inventeur: Heyraud, Marc, 2300 La Chaux-de-Fonds (CH); Grounauer, Pierre-Alain, 1036 Sullens (CH); Kureth, Jean-Bernard, 2300 La Chaux-de-Fonds (CH)
(74) Mandataire: Ganguillet, Cyril
(86) Numéro de dépôt international: CH9800417
(87) Numéro de publication internationale: WO99016343

(56) Documents cités:
- WO-A-91/15988
- DE-C- 2 040 238

## Description

### Domaine technique

La mesure de la pression oculaire (intraocular pressure IOP) est un moyen capital dans le dépistage du glaucome. La présente invention a pour but le perfectionnement des instruments de mesure existants, en particulier des tonomètres d'aplanation et plus particulièrement encore des tonomètres du type Goldmann.

### Etat de la technique

La présente invention se base sur l'instrument de mesure connu, appelé le tonomètre d'aplanation du type Goldmann. Cet appareil que la présente invention permet de rendre plus sûr et dont elle facilite l'usage, est un instrument de mesure mécanique. Un poussoir dont l'extrémité est plane est appliqué directement sur l'oeil du patient, et une certaine force est appliquée mécaniquement. Un objectif permet d'observer la déformation résultante du globe oculaire, sur lequel il se forme une surface plane qui dépend d'une part de la pression interne de l'oeil et d'autre part de la force appliquée sur l'oeil.

Le brevet US 4, 987,899 (A.W. Brown) qui est une référence de base pour tout ce qui concerne la présente description, montre les éléments essentiels du tonomètre d'aplanation du type Goldmann tel qu'il est mondialement connu. A.W. Brown a développé un astucieux système permettant à l'ophtalmologue de lire le tambour sans quitter le prisme d'aplanation des yeux, un capteur de position électromagnétique monté sur l'axe du tambour de mesure formant un signal qui transcrit numériquement la valeur appliquée. A.W. Brown cite en outre dans sa description deux documents antérieurs (US 3,470,736 et 3,070,997) se référant explicitement à la disposition traditionnelle du tonomètre Goldmann.

Ce procédé de mesure consiste à augmenter la force appliquée, jusqu'à ce que la partie déformée du globe oculaire ait atteint une certaine surface. On comprend que les moyens mécaniques mis en oeuvre doivent d'une part permettre de finement doser la force appliquée, et d'autre part permettre une bonne détermination de la surface de référence.

Ces problèmes sont résolus par le tonomètre d'aplanation du type Goldmann fabriqué par la société Haag-Streit: La cornée est aplanie par un poussoir à prisme de dédoublement inséré dans une bague fixée à l'extrémité d'un balancier. La surface antérieure du prisme est plane et ses bords sont légèrement arrondis afin d'éviter tout incident sur la cornée. En tournant le tambour de mesure du tonomètre, on déplace avec une très grande précision des masses placées à l'intérieur du boîtier, ce qui permet d'appliquer une force progressive. On augmente donc la surface aplanie, jusqu'à la valeur fixée de 3,06 mm de diamètre, parfaitement mise en évidence par le prisme. A ce moment le résultat peut être lu sur le tambour de mesure, qui comporte des lignes correspondant à un intervalle de 2 mm Hg. La valeur correcte correspond à la position indiquée sur le tambour en face d'une ligne de référence fixe.

Un des avantages déterminants du tonomètre d'aplanation du type Goldmann est la faible déformation du globe oculaire et donc l'infime quantité de fluide déplacé. De plus la rigidité oculaire et la courbure de la cornée ont peu d'effet sur le résultat de mesure. Le tonomètre d'aplanation Goldmann est peu sensible à la température ou à la pression atmosphérique. Pour toutes ces raisons le tonomètre d'aplanation Goldmann est devenu de facto un standard mondial de mesure.

Toutefois il est connu que ce tonomètre présente un point faible par le fait que, lors de l'opération de mesure, l'ophtalmologue doit lire les graduations sur le tambour et les reporter dans un dossier, alors que la vérification de la surface déformée au moyen du système optique sollicite son attention.

Cette circonstance a conduit certains chercheurs à proposer d'autres appareils de mesure. Ainsi DE 20 40 238 et WO 91/15988 proposent des appareils qui comportent une sonde portable dont les déformations de l'organe sensible sont automatiquement transformées en données correspondant à la surface déformée et à la force de déformation. Toutefois ces appareils n'atteignent pas la fiabilité, la sécurité et la précision du tonomètre de Goldmann.

En ce qui concerne ce dernier appareil, certes le perfectionnement proposé par Brown (US 4, 987, 899) facilite la mesure. D'autre part E. Urinowski et al ont proposé un dispositif permettant de manipuler le tambour du tonomètre avec la même main que le levier de positionnement de celui-ci. Cependant ces améliorations ne valorisent pas l'appareil dans une mesure optimale.

On connaît également des propositions (US 3, 992, 926- Berryhill - et Annales d'oculistique Vol. 185, 1952, p.772 - J. François -) consistant à obtenir une information électronique numérique en utilisant un capteur de pression d'un nouveau type, mais on perd alors les bénéfices de la méthode Goldmann et l'expérience accumulée dans le domaine mécanique. Et il est pratiquement impossible d'équiper les instruments mécaniques déjà utilisés par des milliers d'ophtalmoloques. La transmission de données provenant d'appareils ophtalmiques vers un ordinateur ou une imprimante a été envisagée dans le document US-A-5,528,323.

Ainsi, malgré le besoin manifeste d'une amélioration sur le point mentionné et malgré le marché considérable des tonomètres à aplanation du type Goldmann, il n'a pas été développé de systèmes capables de simplifier l'enregistrement et d'augmenter la fiabilité et la rapidité d'obtention des résultats.

### But et objet de l'invention

Le but de la présente invention est de fournir un dispositif de lecture de la force appliquée qui supprime, pour l'ophtalmologue les difficultés de transcription et les risques d'erreurs que présentent les instruments actuels, tout en conservant les bénéfices de la méthode d'aplanation Goldmann, l'expérience accumulée dans le domaine mécanique et le parc d'instruments actuels.

On a constaté en outre qu'il est souhaitable de transformer l'information ainsi obtenue en un signal électronique numérique, non seulement utilisable directement sur une petite imprimante, mais aussi compatible avec les moyens informatiques dont disposent les médecins actuellement, en particulier les ordinateurs personnels. Cela permet des mesures rapides, un stockage des résultats et toutes les opérations sur les données rendues possibles par l'informatique, en particulier dans le domaine graphique. Il est en effet important de pouvoir répéter rapidement les mesures, les stocker, en tirer des moyennes et des tendances, ou en faire une analyse statistique, toutes choses faciles à réaliser avec un ordinateur personnel. L'instrument de mesure de la pression oculaire selon la présente invention permet d'atteindre ce but d'une manière fiable et rapide, donc économique, et présente en particulier l'avantage de pouvoir être réalisé en adaptant les appareils mécaniques existant.

A cet effet, l'invention concerne un instrument de mesure de la pression oculaire produisant des résultats sous forme électronique, et plus particulièrement un tonomètre d'aplanation de type Goldmann comportant un poussoir s'appliquant directement sur l'oeil du patient, un mécanisme d'application d'une force agissant sur le dit poussoir, un boîtier fixe dans lequel le mécanisme est logé, et un système optique permettant d'observer la déformation du globe oculaire, caractérisé en ce qu'un capteur de position électromécanique est associé au dit mécanisme de manière à former un signal qui transcrit la valeur de la force appliquée.

Le capteur de position peut être agencé pour fournir un signal numérique, en utilisant par exemple un codeur optique incrémental à deux canaux. Alternativement, le capteur de position peut être monté sur l'axe rotatif du tambour de mesure ou on peut utiliser un capteur de position linéaire monté sur une pièce mobile du dispositif mécanique permettant d'ajuster la force appliquée.

L'instrument de mesure selon l'invention peut comporter un détecteur d'une position de référence, pouvant être monté sur une pièce mobile à l'intérieur du dispositif mécanique ou intégré au capteur de position sur l'axe rotatif du tambour de réglage. Alternativement, le détecteur de la position de référence peut être un système optique, magnétique, inductif ou capacitif déclenché sans contact par le mouvement d'une pièce mobile à l'intérieur du dispositif mécanique.

La transmission des données résultant de la mesure à un ordinateur ou à tout autre système de traitement peut s'effectuer sans fil par ondes radio, par ondes infrarouges ou par ondes sonores ou ultrasonores ou par un système optique. Le circuit de transmission peut également être basé sur le circuit d'une souris d'ordinateur.

L'instrument de mesure selon l'invention peut comporter un système de décodage redondant augmentant la fiabilité des informations transmises.

La connexion du tonomètre à l'ordinateur peut se faire par câble. Mais il peut alors être difficile d'équiper les instruments mécaniques déjà utilisés par des milliers d'ophtalmologues sans l'intervention d'un monteur. Une nouvelle disposition du cabinet de l'ophtalmologue est parfois nécessaire à cause des câbles. De plus les ophtalmologues ne souhaitent pas voir leurs cabinets encombrés de câbles électriques, qui risquent d'être accrochés par leurs patients. Dans de tels cas le signal numérique est un avantage car il peut être facilement transmis par diverses techniques, telles que ondes radio ou infrarouges, ultrasons ou toute autre méthode fiable de transmission sans fil à courte distance.
Un résultat comparable peut être obtenu dans d'autres domaines de la mesure manométrique, par exemple dans la mesure veinomanométrique.

L'invention repose sur une combinaison nouvelle d'éléments connus dans différentes branches de la technique, à savoir le tonomètre mécanique du domaine de la mesure médicale, et le capteur de position, du domaine du réglage des moteurs électriques et des machines-outils. Le capteur dit électromécanique (on devrait dire mécano-électrique) fait correspondre à une position mécanique un signal électrique. Citons par exemple le capteur optique incrémental ou absolu, le résolveur, le capteur capacitif, le potentiomètre. L'intégration d'un capteur de position au tonomètre lui apporte une grande amélioration d'usage, le rendant par là même plus fiable et permettant plus d'examens pour un temps donné. Cette solution permet de résoudre les problème évoqués ci-dessus, avec des moyens plus simples que ceux utilisés par Berryhill ou J.François. Lorsqu'une transmission sans fil est souhaitable, les signaux électriques ainsi obtenus sont transmis à un ordinateur en utilisant un circuit semblable à celui qu'on trouve dans une souris d'ordinateur de type Logitec "Cordless MouseMan Pro(R)", amélioré pour atteindre les objectifs de fiabilité correspondant aux attentes des ophtalmologues.

### Description

La description qui suit, donnée à titre d'exemple, se réfère au dessin sur lequel:
la figure 1 est une vue arrière du boîtier ouvert d'une première forme d'exécution de l'instrument de mesure selon l'invention,
la figure 2 est une vue latérale de l'instrument de la figure 1,
la figure 3 est une vue plus détaillée du capteur optique incrémental de l'instrument de la figure 1,
la figure 4 est une vue arrière du boîtier ouvert d'une seconde forme d'exécution de l'instrument de mesure selon l'invention, et
la figure 5 est une vue latérale de l'instrument de la figure 4.

Dans une forme préférée de l'invention, un disque comportant des fentes situées à un angle constant sur la périphérie à été introduit sur l'axe du tambour de mesure, et son mouvement module un signal lumineux projeté sur un élément électronique photosensible, selon un procédé bien connu dans le domaine du réglage des moteurs à courant continu et que l'on nomme généralement "capteur optique incrémental" (voir par exemple M. Heyraud, La Revue Polytechnique No 1478, 8/86, p.763). Ces capteurs sont disponibles sur le marché avec deux éléments photosensibles qui permettent une discrimination du sens de rotation. II est aussi très courant d'avoir une fente unique sur un diamètre particulier, et qui sert de position de référence, appelée index. Cette position de référence peut aussi être obtenue avec d'autres dispositifs mécaniques, optiques, capacitifs, inductifs, etc., qui donne un signal pour un seul angle de rotation du poulet, ou pour une position fixe des masses mobiles du tonomètre. Un circuit électronique permet le décodage et le comptage des signaux modulés transmis par l'élément électronique photosensible. Les signaux électriques ainsi obtenus peuvent être transmis à un ordinateur ou à n'importe quel autre système de traitement des données numériques.

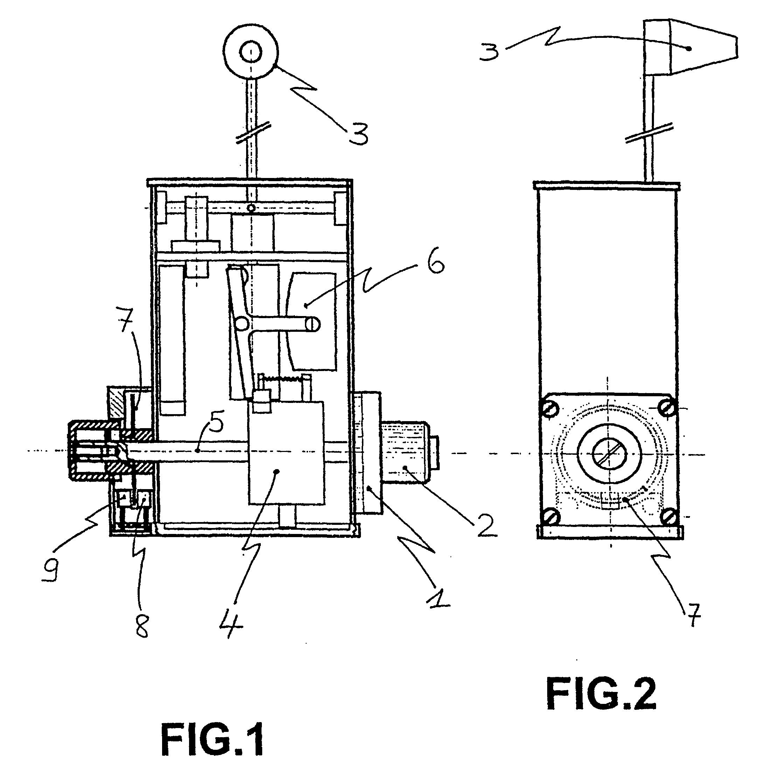

Selon une première forme d'exécution, représentée sur les figures 1 et 2, l'instrument de mesure selon l'invention est réalisé à partir d'un tonomètre à aplanation disponible sur le marché. La figure 1 est une vue du boîtier ouvert du tonomètre. On a représenté de manière simplifiée le tambour de mesure 1, avec le poulet 2 manipulé par l'ophtalmologue durant la mesure, le poussoir plan 3, la masse 4 déplacée le long de l'axe 5 supportant le tambour, ainsi que le dispositif mécanique 6 permettant le réglage de la force appliquée. On a aussi représenté sur la figure 1 le système de capteur incrémental optique monté sur le côté du boîtier opposé au tambour, mais qui peut être monté dans le tambour de mesure. Le disque à fentes 7 rigidement fixé sur l'axe du tambour 5 passe entre un émetteur électronique de lumière (une photodiode dans le mode d'exécution préféré) 8 et un récepteur électronique photosensible (quatre phototransistors dans le mode d'exécution préféré) 9. Le faisceau lumineux de l'émetteur électronique de lumière 8 est modulé par la rotation du disque 7, donc par la rotation du tambour de mesure, puis capté et transformé en signal électronique de même modulation par le récepteur électronique photosensible 9. Dans la forme d'exécution préférée, le circuit électronique de décodage et comptage est intégré dans le même boîtier que les phototransistors 9. Pour la remise à zéro, l'étalonnage et la maintenance facile du tonomètre, la forme d'exécution préférée comporte aussi des commutateurs dit "de fin de course" et bien connus dans l'industrie des machines, non représentés sur le dessin, qui sont actionnés par le déplacement de la pièce mécanique 4. Une autre forme d'exécution serait réalisée en utilisant la pièce mécanique 4 comme support d'un capteur linéaire optique, magnétique ou capacitif.

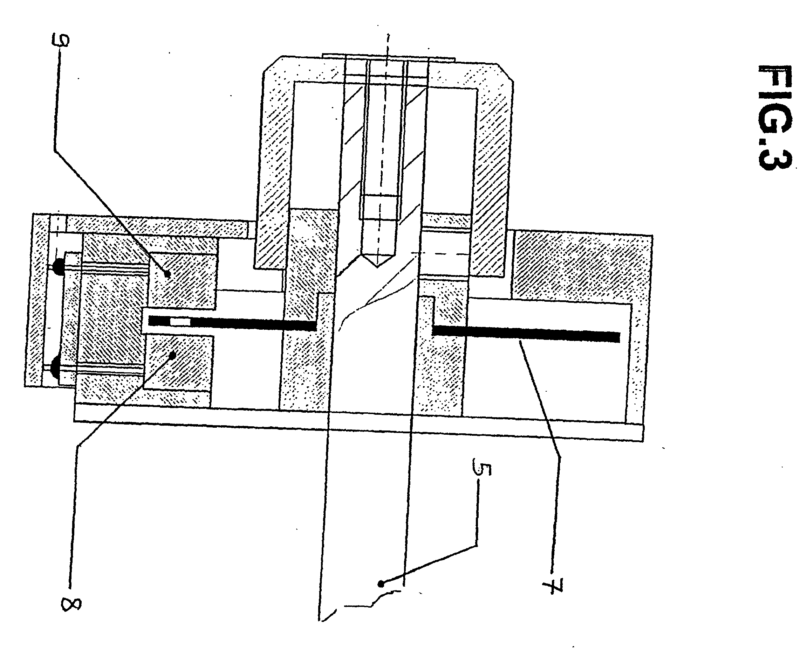

La figure 3 est une vue plus détaillée du capteur optique incrémental.

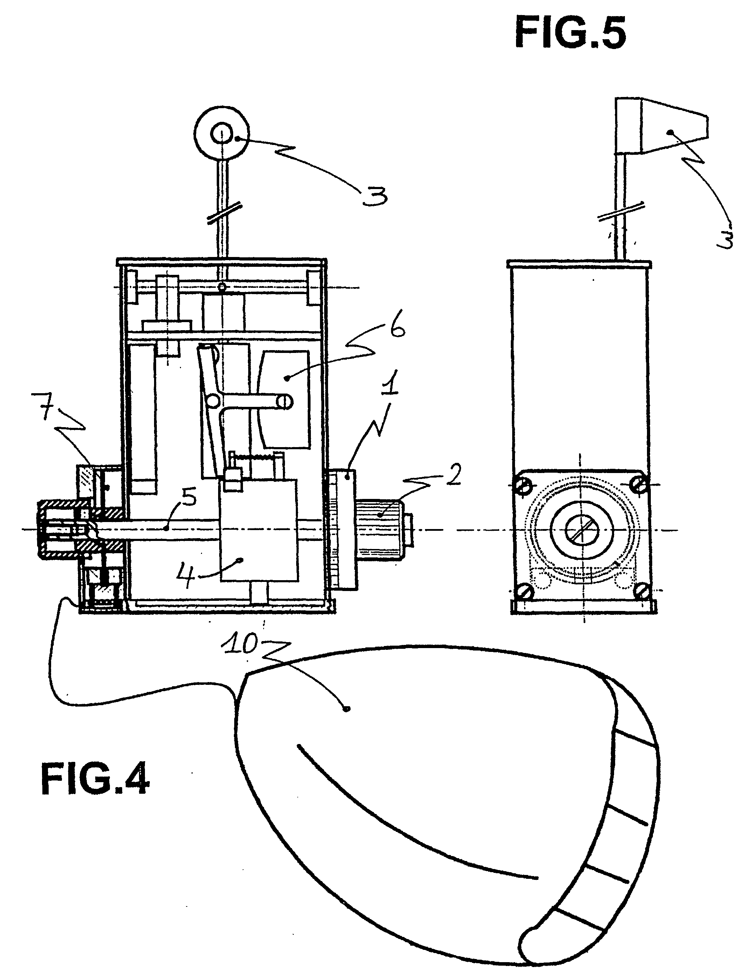

Les figures 4 et 5 illustrent une forme d'exécution de l'instrument de mesure selon l'invention où les signaux électriques sont transmis à un ordinateur en utilisant un circuit semblable à celui qu'on trouve dans une souris d'ordinateur 10, par exemple de type Logitec "Cordless MouseMan Pro(R)" amélioré pour atteindre les objectifs de fiabilité correspondant aux attentes des ophtalmologues.

A cet égard, les mesures qui peuvent être prises pour atteindre ces objectifs de fiabilité sont les suivantes:
- redondance: la "souris" dispose de deux canaux, correspondant aux deux directions de déplacement sur un plan. Comme la présente invention n'utilise qu'un seul signal, on pourrait se contenter d'un seul canal. Mais en transmettant sur les deux canaux en parallèle, et en comparant les signaux reçus par l'ordinateur dans l'ordinateur lui-même, on introduit une redondance qui augmente notablement la fiabilité de la transmission.
- transmission en continu: la transmission rapide et continue veut dire qu'il est possible de vérifier sur plusieurs envois la valeur transmise. Cette redondance temporelle augmente aussi notablement la fiabilité.
- codage et décodage: à l'aide de circuits de codage à l'émission et de décodage à la réception disponibles sur le marché, il est possible de réduire les risques de transmission erronée.

Alors que ces mesures permettant d'augmenter la fiabilité sont indispensables lorsqu'on utilise une "souris", elles sont souhaitables avec d'autres systèmes. Elles peuvent donc être prévues ensemble ou séparément dans des formes d'exécution préférées de l'invention.

## Revendications

1. Tonomètre d'applanation de type Goldmann comportant un poussoir (3) s'appliquant directement sur l'oeil du patient, un mécanisme d'application d'une force (6, 4, 5) agissant sur le dit poussoir, un boîtier fixe dans lequel le mécanisme est logé, un système optique permettant d'observer la déformation du globe oculaire, un capteur de position électromécanique (7, 8, 9) associé au dit mécanisme (6, 4, 5) et un moyen électronique de traitement de données associé au capteur et traitant un signal formé par le capteur de position, **caractérisé en ce qu'**un circuit de transmission est prévu pour transmettre le dit signal, du capteur au moyen de traitement de données, et **en ce que** ce circuit de transmission est basé sur celui d'une souris d'ordinateur disposant de deux canaux qui sont utilisés en parallèle de manière à introduire une redondance augmentant la fiabilité de la transmission.

2. Tonomètre selon la revendication 1, **caractérisé en ce que** le moyen électronique de traitement de données est agencé pour transformer l'information contenue dans le signal du capteur en une donnée transcrivant la valeur de la force appliquée, apte à être imprimée ou stockée, ou compatible avec des moyens informatiques tels que les ordinateurs personnels.

3. Tonomètre selon la revendication 1 ou la revendication 2, **caractérisé en ce que** le capteur de position est agencé pour fournir le dit signal sous forme numérique.

4. Tonomètre selon l'une des revendications précédentes, **caractérisé en ce que** le capteur de position est monté de manière à capter la position instantanée d'une pièce mobile du mécanisme se déplaçant avec des moyens d'application de force ajustables selon la force appliquée.

5. Tonomètre selon la revendication 4, **caractérisé en ce que** la dite pièce mobile du mécanisme est un axe rotatif (5) d'un tambour de mesure (1, 2).

6. Tonomètre selon la revendication 4, **caractérisé en ce que** le capteur est un codeur optique incrémental à deux canaux (7, 8, 9).

7. Tonomètre selon la revendication 4, **caractérisé en ce que** le capteur comporte un détecteur d'une position de référence.

8. Tonomètre selon la revendication 7, **caractérisé en ce que** le détecteur d'une position de référence est un système optique, magnétique, inductif ou capacitif, déclenché sans contact par le mouvement d'une pièce mobile à l'intérieur du mécanisme d'application progressive de la force.

9. Tonomètre selon l'une quelconque des revendications précédentes, **caractérisé en ce que** la transmission du signal se fait sans fil.

10. Tonomètre selon la revendication 9, **caractérisé en ce que** la transmission du signal s'effectue par ondes radio, infrarouges, sonores, ultrasonores ou de lumière visible.

11. Tonomètre selon l'une des revendications précédentes, **caractérisé en ce que** le moyen de traitement des données est un ordinateur.

## Patentansprüche

1. Applanationstonometer nach Goldmann umfassend einen Druckkörper (3), der direkt auf das Auge des Patienten aufgebracht wird, einen Mechanismus zum Aufbringen einer an diesem Druckkörper angreifenden Kraft (6, 4, 5), ein stationäres Gehäuse, in dem der Mechanismus untergebracht ist, ein optisches System zum Beobachten der Deformation des Augapfels, ein diesem Mechanismus zugeordneter elektromechanischer Positionssensor (7, 8, 9) und ein elektronisches Mittel zur Datenverarbeitung, das diesem Sensor (6, 4, 5) zugeordnet ist und ein vom Positionssensor gebildetes Signal verarbeitet, **dadurch gekennzeichnet, dass** eine Übertragungsschaltung zur Übertragung dieses Signals vom Sensor zum Datenverarbeitungsmittel vorgesehen ist und dass diese Übertragungsschaltung auf der einer Computermaus beruht und über zwei Kanäle verfügt, die parallel verwendet werden, um somit eine die Zuverlässigkeit der Übertragung erhöhende Redundanz einzuführen.

2. Tonometer nach Anspruch 1, **dadurch gekennzeichnet, dass** das elektronische Mittel zur Datenverarbeitung dazu ausgebildet ist, die im Sensorsignal enthaltene Information in Daten umzuwandeln, die den Wert der aufgewendeten Kraft übertragen und die gedruckt oder gespeichert werden können, oder mit Mitteln zur elektronischen Datenverarbeitung, wie z.B. PC's, kompatibel sind.

3. Tonometer nach Anspruch 1 oder 2, **dadurch gekennzeichnet, dass** der Positionssensor eingerichtet ist, das Signal in numerischer Form zu liefern.

4. Tonometer nach einem der vorstehenden Ansprüche, **dadurch gekennzeichnet, dass** der Positionssensor derart montiert ist, dass er die momentane Position eines beweglichen Teils des Mechanismus erfasst, wobei sich dieses Teil mit Mitteln zum Aufbringen einer Kraft bewegt, die je nach der aufgewendeten Kraft einstellbar sind.

5. Tonometer nach Anspruch 4, **dadurch gekennzeichnet, dass** das bewegliche Teil des Mechanismus eine Drehachse (5) einer Messtrommel (1, 2) ist.

6. Tonometer nach Anspruch 4, **dadurch gekennzeichnet, dass** der Sensor ein optischer Inkrementalkodierer mit zwei Kanälen (7, 8, 9) ist.

7. Tonometer nach Anspruch 4, **dadurch gekennzeichnet, dass** der Sensor einen Detektor zur Ermittlung einer Sollposition aufweist.

8. Tonometer nach Anspruch 7, **dadurch gekennzeichnet, dass** der Detektor zur Ermittlung der Sollposition ein optisches, magnetisches, induktives oder kapazitives System ist, das berührungslos durch die Bewegung eines beweglichen Teils im Inneren des Mechanismus zum Aufbringen einer allmählich ansteigenden Kraft ausgelöst wird.

9. Tonometer nach einem der vorstehenden Ansprüche, **dadurch gekennzeichnet, dass** die Signalübertragung kabellos erfolgt.

10. Tonometer nach Anspruch 9, **dadurch gekennzeichnet, dass** das Signal über Radio-, Infrarot-, Schall-, Ultraschallwellen oder über sichtbares Licht übertragen wird.

11. Tonometer nach einem der vorstehenden Ansprüche, **dadurch gekennzeichnet, dass** das Mittel zur Datenverarbeitung ein Rechner ist.

## Claims

1. An applanation tonometer of the Goldman type comprising a pusher (3) to be applied directly to the eye of the patient, a force application mechanism (6, 4, 5) acting on the said pusher, a fixed casing in which the mechanism is housed, an optical system for observing the deformation of the eyeball, an electromechanical position sensor (7, 8, 9) associated with the said mechanism (6, 4, 5) and an electronic data processing means associated with the sensor and processing a signal formed by the position sensor, **characterised in that** a transmission circuit is provided for transmitting the said signal from the sensor to the data processing means, and **in that** this transmission circuit is based on that of a computer mouse having two channels which are used in parallel so as to introduce redundancy increasing the reliability of the transmission.

2. A tonometer according to claim 1, **characterised in that** the electronic data processing means is arranged so as to transform the information contained in the signal from the sensor into a data item transcribing the value of the force applied, able to be printed or stored, or compatible with data processing means such as personal computers.

3. A tonometer according to claim 1 or claim 2, **characterised in that** the position sensor is arranged to supply the said signal in digital form.

4. A tonometer according to one of the preceding claims, **characterised in that** the position sensor is mounted so as to capture the instantaneous position of a moving part of the mechanism moving with force application means adjustable according to the force applied.

5. A tonometer according to claim 4, **characterised in that** the said moving part of the mechanism is a rotating spindle (5) of a measuring drum (1, 2).

6. A tonometer according to claim 4, **characterised in that** the sensor is an incremental optical coder with two channels (7, 8, 9).

7. A tonometer according to claim 4, **characterised in that** the sensor comprises a reference position detector.

8. A tonometer according to claim 7, **characterised in that** the detector of a reference position is a magnetic, inductive or capacitive optical system, triggered without contact by the movement of a moving part inside the progressive force application mechanism.

9. A tonometer according to any one of the preceding claims, **characterised in that** the transmission of the signal takes place without any wire.

10. A tonometer according to claim 9, **characterised in that** the signal is transmitted by radio, infrared, sound, ultrasound or visible light waves.

11. A tonometer according to one of the preceding claims, **characterised in that** the data processing means is a computer.
